Europäisches Patentamt

(19) European Patent Office

Office européen des brevets

(11) Publication number: **0 338 057 B1**

(12) **EUROPEAN PATENT SPECIFICATION**

(45) Date of publication of patent specification: **15.12.93**　　(51) Int. Cl.⁵: **C12N 9/10**, C12P 19/18

(21) Application number: **88909307.6**

(22) Date of filing: **14.10.88**

(86) International application number:
**PCT/DK88/00168**

(87) International publication number:
**WO 89/03421 (20.04.89 89/09)**

(54) **THERMOSTABLE CYCLODEXTRIN GLYCOSYL TRANSFERASE, ITS PRODUCTION AND USE.**

(30) Priority: **15.10.87 US 108688**
**15.10.87 US 108469**

(43) Date of publication of application:
**25.10.89 Bulletin 89/43**

(45) Publication of the grant of the patent:
**15.12.93 Bulletin 93/50**

(84) Designated Contracting States:
**AT BE CH DE FR GB IT LI LU NL SE**

(56) References cited:
**DE-A- 2 447 132**
**US-A- 4 628 028**

**Patent abstract of Japan, vol. 9, n 271, C-311, abstracts of JP 60-120984, publ. 1985-06-28**

**Chemical abstracts, vol. 108 (1988) abstract n 163779y, Prog. Biotechnol. 1987, 3 (Ind. Polysaccharides), 81-93 (Eng.)**

(73) Proprietor: **NOVO NORDISK A/S**
**Novo Allé**
**DK-2880 Bagsvaerd(DK)**

(72) Inventor: **STARNES, Robert, L.**
**140 Captain Thomas Blvd., no. 401**
**West Haven, CT 06516(US)**
Inventor: **TRACKMAN, Philip, C.**
**1750 Central Avenue**
**Needham, MA 02192(US)**
Inventor: **KATKOCIN, Dennis, M.**
**32 Sunswept Drive**
**New Fairfield, CT 06812(US)**

(74) Representative: **Knudsen, Sten Lottrup et al**
**c/o Novo Nordisk A/S,**
**Patent Department,**
**Novo Allé**
**DK-2880 Bagsvaerd (DK)**

Chemical abstracts, vol. 109 (1988), abstract n 26564v, Appl.Microbiol. Biotechnol. 1988, 28(4-5), 377-9 (Eng)

Chemical abstracts, vol. 98 (1983) abstract n 85257k, Biotechnol Bioeng. Symp. 1982, 12 (Symp. Biotechnol. Energy Prod. Conserv. 4th), 461-7 (Eng)

## Description

### TECHNICAL FIELD

This invention relates to a cyclodextrin glycosyl transferase, to a method for producing it, to a microbial strain capable of producing it, and to its use in starch liquefaction and in cyclodextrin production.

### BACKGROUND ART

Cyclodextrin glycosyl transferase (1,4-$\alpha$-D-glucan 4-$\alpha$-D-(1,4-$\alpha$-D-glucano)-transferase, E.C. 2.4.1.19), hereinafter termed cyclodextrin glycosyl transferase or CGTase, is an enzyme characterized by its ability to form cyclodextrins by cyclization of starch or starch hydrolyzate.

Thus, the enzymatic cyclization reactions produce cyclodextrins (abbreviated as CD and also known as Schardinger dextrins), such being cyclic molecules comprised of six, seven or eight $\alpha$-D-glucopyranose residues linked by $\alpha$-1,4 bonds. They are known as $\alpha$-, $\beta$-, or gamma-cyclodextrins depending on the number of glucose residues, 6, 7 or 8, respectively.

Typically, cyclodextrins have been prepared heretofore by variations of the method described by E.B. Tilden and C.S. Hudson (J. American Chemical Society) 64:1432[1942], which method involves treating liquefied starch with a cyclodextrin glycosyl transferase (CGTase) enzyme from Bacillus macerans. All variations of this process have a number of disadvantages. First, since the CGTase is not sufficiently thermostable to be used above the gelatinization temperature of starch, the starch must be pretreated, e.g., with an $\alpha$-amylase, to solubilize the starch. It is important that the starch be liquefied to a relatively low DE (Dextrose Equivalent), so after conduct of the starch liquefaction process, the treating agent, normally an $\alpha$-amylase, must be inactivated, to obtain good cyclodextrin yield. Second, the Bacillus macerans CGTase is not sufficiently stable to be used at elevated temperatures, and consequently, the enzymatic cyclodextrinization process is carried out at about 50°C, where it is subject to possible microbial contamination. Third, conversion of starch to cyclodextrins (at 50°C, pH 7.0) by the Bacillus macerans CGTase requires extended reaction time, before reasonable yields are achieved.

The CGTase enzymes heretofore known to the art are produced by such microorganisms as Bacillus macerans, Bacillus circulans, Bacillus stearothermophilus, Bacillus megaterium, Bacillus ohbensis, alkalophilic Bacillus sp., Micrococcus luteus, Micrococcus varians, and Klebsiella pneumoniae. However, none of the CGTase enzymes produced by these microorganisms are sufficiently stable at above 60°C for use to produce cyclodextrins at temperatures sufficiently elevated to avoid possible microbial contamination.

Enzymatic liquefaction of aqueous starch slurry is widely practiced as the first step in converting starch to dextrose (glucose). To a great extent the starch industry has adopted the liquefaction process of US 3,921,590. Typical conditions are jet cooking at 105°C for 5 minutes, followed by a 90 minute hold at 95°C, at a starch concentration of 35% DS (dry substance), by weight. The enzyme used in this process is Termamyl ™ (product of Novo Industri A/S), an $\alpha$-amylase from Bacillus licheniformis. Liquefaction is performed at pH about 6.0, followed by saccharification with glucoamylase at a pH of approx 4.5-5.0.

The art has long sought starch liquefaction enzymes capable of liquefying at pH 4.5, in order to eliminate the need for intermediate pH adjustment. $\alpha$-amylase from Bacillus stearothermophilus has been suggested for this purpose, but data in this specification show that it does not liquefy well at pH as low as 4.5. US 4,578,532 and US 4,613,570 disclose aciduric $\alpha$-amylases from Clostridium, but data in said patents show that their stability at 100°C or above at pH 4.5 is insufficient.

### OBJECT OF THE INVENTION

It is an object of this invention to provide a cyclodextrin glycosyl transferase with sufficient heat stability to be used for CD production at 60°C or higher, where the risk of microbial infection is slight, and even to be used for starch liquefaction above 90°C, where the starch is fully gelatinized.

It is also an object of the invention to provide an enzyme capable of liquefying starch at pH 4.5 and a temperature above 100°.

Other objects of the invention are the provision of a method of producing said thermostable enzyme and a microbial strain capable of producing it. It is a further object to provide a process using said enzyme to produce CD at 60°C or higher and a process using said enzyme for starch liquefaction at a pH around 4.5-5.0.

3

## STATEMENT OF THE INVENTION

The inventors have isolated a number of thermophilic obligate anaerobic strains that produce CGTases of surprising heat stability.

Accordingly, in its first aspect the invention provides a cyclodextrin glycosyl transferase, characterized in that it is native to a strain of Thermoanaerobacter or Thermoanaerobium, has a temperature optimum measured at pH 5.0 of about 95°C; a pH optimum of about 5.0; and a residual activity after 40 minutes incubation at 80°C and pH 5.0 of about 95% in the absence of starch and Ca++.

A second aspect of the invention provides a method for producing a cyclodextrin glycosyl transferase (CGTase) comprising cultivation of a CGTase producing strain of Thermoanaerobacter or Thermoanaerobium under anaerobic conditions, or cultivation of a transformed host organism containing the appropriate genetic information therefrom under aerobic conditions, in a suitable nutrient containing medium and thereafter recovering CGTase from the fermentation medium.

A third aspect of the invention provides a biologically pure culture of a strain of Thermoanaerobacter or Thermoanaerobium, characterized by the ability to produce cyclodextrin glycosyl transferase, and by being non-motile.

A fourth aspect of the invention provides a starch liquefaction process which comprises subjecting an aqueous starch slurry to enzymatic liquefaction in the presence of said cyclodextrin glycosyl transferase at a pH in the range of about 4.0 to 5.5 preferably at a temperature exceeding about 100°C.

A fifth aspect of the invention provides a process for producing cyclodextrin which comprises enzymatically treating a starch hydrolysate solution with said cyclodextrin glycosyl transferase, at a temperature of above 60°C and thereafter recovering a cyclodextrin product from the reaction mixture.

Finally, a sixth aspect of the invention provides a process for producing cyclodextrin which comprises enzymatically treating an aqueous starch slurry with the cyclodextrin glycosyl transferase of Claim 1 at a temperature of above about 100°C and at a pH in the range of 4.0 - 5.5, preferably essentially without addition of a calcium salt, thereafter holding the resulting syrup at a temperature in the range of 80° - 90°C for not more than about 28 hours, the syrup being in the range of 20 - 30 DS during at least part of said hold period, and then recovering a cyclodextrin product from the reaction mixture.

## DETAILED EXPLANATION OF THE INVENTION

### Microorganism

The microorganisms of the invention are thermophilic obligate anaerobic bacteria belonging to the genus Thermoanaerobacter (J. Wiegel and L.G. Ljungdahl, Arch. Mirobiol. (1981) 128: 343-348) or the genus Thermoanaerobium (J.G. Zeikus et al., Arch. Microbiol., 122, 41-48 (1979). The taxonomy for these genera is not established, and it is considered likely that the two genera could properly be classified as one and the same genus, since they are so similar that even an expert in this field cannot differentiate them.

In contrast to known strains of Thermoanaerobacter and Thermoanaerobium, the microbial strains of the invention are characterized by the ability to produce CGTase and by being non-motile. Some strains of the invention are also indole positive, in contrast to known strains. Known strains T. ethanolicus DSM 3389 and T. finii DSM 2246 were fouund not to be producers of thermostable CGTase.

8 strains were isolated by the inventors and were deposited at the American Type Culture Collection (ATCC) and the National Collections of Industrial Marine Bacteria (NCIMB) for patenting purposes under the terms of the Budapest Treaty, as follows:

|  | Deposit No. | Deposit Date | Depositor's Reference |
|---|---|---|---|
| 1. | ATCC 53,627 | June 3, 1987 | ANO-15-7-5A2-70 |
| 2. | NCIB 40,053 | October 6, 1988 | ANO-16-7-2A-70 |
| 3. | NCIB 40,054 | October 6, 1988 | ANO-16-7-4A-70 |
| 4. | NCIB 40,055 | October 6, 1988 | ANO-36-7-1 |
| 5. | NCIB 40,056 | October 6, 1988 | ANO-38-7-1 |
| 6. | NCIB 40,057 | October 6, 1988 | ANO-44-5-1-55 |
| 7. | NCIB 40,058 | October 6, 1988 | ANO-51-7-1-70 |
| 8. | NCIB 40,059 | October 6, 1988 | ANO-55-7-1-70 |

Mutants and variants of the above strains are also within the scope of the invention.

4

These 8 strains were al classified by NCIMB, Scotland, as <u>Thermoanaerobacter</u> sp. or <u>Ther-moanaerobium</u> sp., the genus being unresolved. Further taxonomic data are given below:

| Strain No. | 1 | 2 | 3 | 4 | 5 | 6 | 7 | 8 |
|---|---|---|---|---|---|---|---|---|
| °C incubation | 55 | 60 | 60 | 60 | 60 | 60 | 60 | 60 |
| Cell morphology | | | (a) | | | | (b) | |
| Gram | – | – | – | – | – | – | – | var. |
| Spores | – | – | – | – | – | – | – | – |
| Motility | – | – | – | – | – | – | – | – |
| Colonial Morphology | (c) | (d) | | (d) | | (d) | | (d) |
| Growth   30°C | (e) | | | | | | | |
|          37°C | ND | ND | | + | | | | |
|          50°C | + | | | | | | | |
| Viscous in KOH test | + | + | + | + | + | + | + | + |
| Growth Glucose Ye | (+) | + | + | + | + | – | – | + |
| Growth TYG | + | ND | ND | + | ND | – | – | + |
| Catalase | – | – | – | – | – | – | – | – |
| Oxidase, Kovacs P-W-S | (f) | – | – | – | – | – | – | – |
| Chloramphenicol sensitivity | + | | | | | | | |
| Haemolysis on horse blood agar | – | | | | | | | |
| Litmus milk reduction PNPG | + | | | | | | | |
| $H_2S$ production | + | | | | | | | |

**See notes (a) - (f) on next page.**

**Notes:**

(a)  Granular rods of varying length, in chains.

(b)  Regular rods, 'granular' staining, singly and in short chains.

(c)  (starch agar, 3 days): round, regular, entire, smooth, low (?), convex (?), opaque, yellowish-buff, 2.5 mm diameter.

(d)  (R.C.M., 3 days): round, regular, entire, smooth, shiny, translucent, flat, white, 3 mm diameter.

(e)  + (slow 7 days).

(f)  Peptone Water Sugar: No acid or gas.

ND  Not determined

6

## API 20A Anaerobic Test
## 24 hours 60°C

| Strain No. | 1 | 2 | 3 | 4 | 5 | 6 | 7 | 8 |
|---|---|---|---|---|---|---|---|---|
| Indole | + | | | − | − | − | | |
| Urease | | NC | NC | − | − | − | NC | NC |
| Acid from: | | | | | | | | |
| Glucose | | | | − | − | − | | |
| Mannitol | | | | − | − | − | | |
| Lactose | | | | − | − | − | | |
| Sucrose | | | | + | − | − | | |
| Maltose | | | | + | − | + | | |
| Salicin | | | | + | − | − | | |
| Xylose | | | | + | + | + | | |
| Arabinose | | | | − | − | + | | |
| Glycerol | | | | − | − | − | | |
| Cellobiose | | | | ± | − | + | | |
| Mannose | | | | + | − | + | | |
| Melezitose | | | | − | − | − | | |
| Raffinose | | | | − | − | − | | |
| Sorbitol | | | | − | − | − | | |
| Rhamnose | | | | − | − | + | | |
| Trehalose | | | | ± | (+) | + | | |
| Gelatinase* | | | | − | + | + | | |
| Aesculin hydrolysis | | | | + | + | + | | |

NC   No change after incubation

±   Trace reaction

*   May be artefact caused by high temperature

**Production of CGTase**

The preparation of the CGTase enzyme may be accomplished by culturing a microbial strain of the invention (for example ATCC 53,627) under anaerobic conditions in a medium which contains maltodextrin

7

as the carbon source, yeast extract, and mineral solutions. The optimum pH and temperature for production of the CGTase is pH 7.0 and 67°C. The enzyme is excreted into the fermentation medium indicating it is an extracellular enzyme.

Alternatively, CGTase of the invention can be produced by aerobic cultivation of a transformant containing the appropriate genetic information. In general, this method of production will comprise the following steps:

(a) providing a suitable recombinant DNA cloning vector comprising DNA sequences encoding functions facilitating gene expression and a DNA sequence encoding the CGTase of a Thermoanaerobacter or Thermoanaerobium strain;

(b) transforming a suitable host organism with the cloning vector from step (a);

(c) cultivating the transformed host under aerobic conditions in a suitable nutrient containing medium and thereafter recovering CGTase from the medium

Examples of suitable host organisms are strains of Escherichia, Streptomyces, Bacillus or Aspergillus, preferably a strain of E.coli, B. subtilis, B. licheniformis or A. oryzae.

The CGTase can be recovered by removing the cells from the fermentation medium and then concentrating the broth, e.g. by ultrafiltration.

## Purification of CGTase

For characterization purposes, purification of the crude CGTase preparation from ATCC 53,627 to homogeneity was achieved by DEAE-Sepharose chromatography, Chromatofocusing[R],and acarbose-Sepharose affinity chromatography. Three components designated I, II and III were purified. Only one CGTase component was found in the crude preparations from the other strains, based on SDS-polyacrylamide gel electrophoresis.

## Thermostability of CGTase

CGTases of the invention are characterized by thermostability far superior to prior-art enzymes. After incubation in 5% Lintner starch - 0.1M sodium acetate pH 5.0 (50 ppm $Ca^{++}$) - for 50 minutes at 95°C, CGTase of the invention (ATCC 53,627) retains nearly 100% of its activity.

Figure 3 shows the residual activity of crude CGTase from ATCC 53,627 after 40 minutes incubation at various temperatures at pH 5.0 in the absence of substrate and $Ca^{++}$. As shown, it retains approx. 95% of its activity at 80°C at these conditions. For comparison, data for two prior-art liquefying enzymes are also shown: α-amylase from Bacillus licheniformis (Termamyl[TM]) and α-amylase from Bacillus stearothermophilus.

Components I, II and III have similar thermostability as the crude CGTase of ATCC 53,627. For comparison, the Bacillus macerans CGTase is reported to be stable only at temperatures below 50°C and to lose activity rapidly at above 50°C (Stavn, A. and Granum, P.E. in Carbohydrate Research, 75 [1979] 243).

## Characterization of CGTase

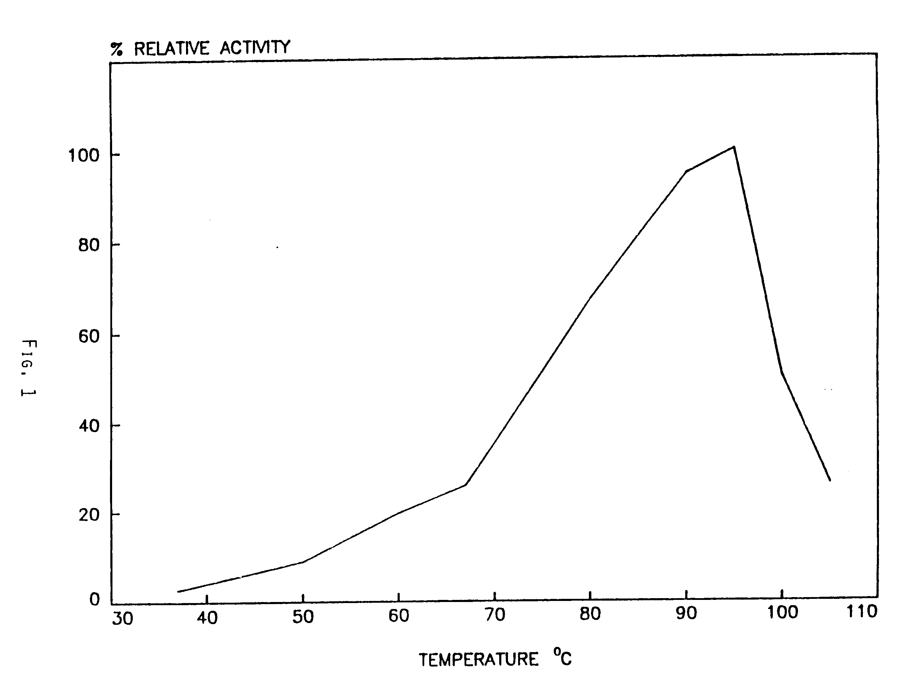

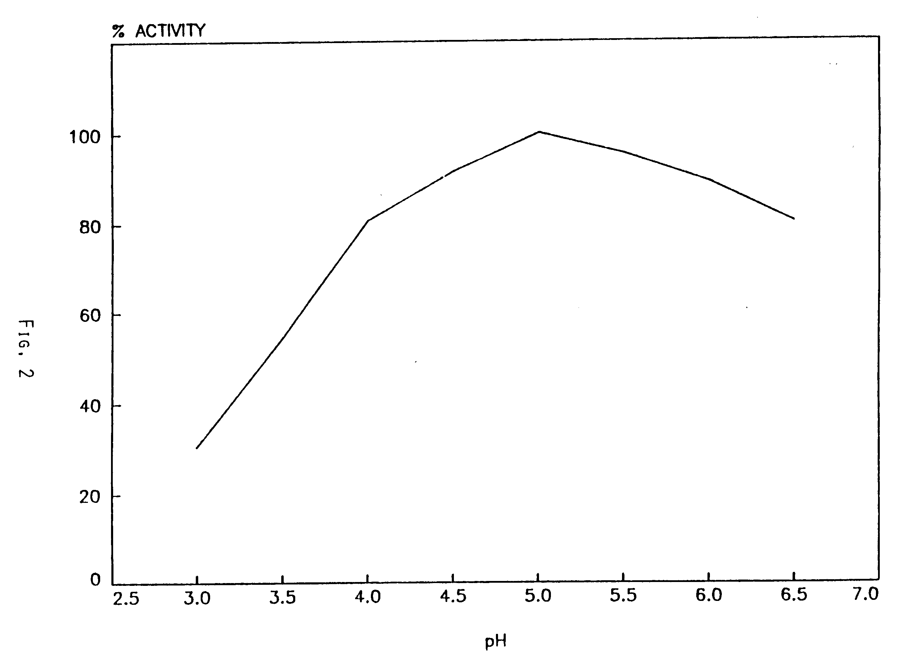

Temperature Optimum. The effect of temperature on CGTase activity was determined. The CGTase from ATCC 53,627 possesses a temperature optimum at 95°C at pH 5.0 in 0.1M sodium acetate - 100 ppm $Ca^{++}$ (see Figure 1). This optimum is in contrast to that of the Bacillus macerans CGTase which is reported to be 55°C at pH 6.0 (Stavn, A. and Granum, P.E. in Carbohydrate Research, 75 [1979] 243).

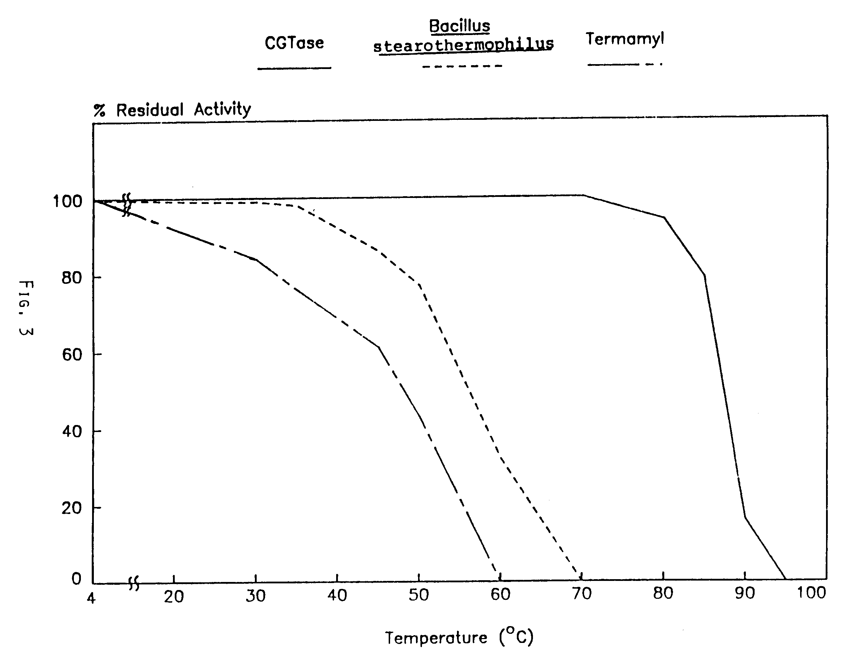

pH Optimum. The effect of pH on CGTase activity was examined at 60°C. The pH optimum of the CGTase from ATCC 53,627 is 5.0 with broad activity in the acidic region when tested in a citratephosphate-0.5% Lintner starch-100 ppm $Ca^{++}$ buffer system (see Figure 2). This value is similar to the pH optimum of 5.2-5.7 reported for Bacillus macerans CGTase (Stavn, A. and Granum, P.E. in Carbohydrate Research, 75 [1979] 243).

Molecular Weight. The molecular weights of the CGTases, determined by SDS-polyacrylamide gel electrophoresis followed by a 0.8% Lintner starch-iodine Gelrite[R] overlay at pH 6.0, 55°C, were as follows:

| ATCC 53,627 I | 117,000 Daltons |
|---|---|
| ATCC 53,627 II | 110,000 - |
| ATCC 53,627 III | 108,000 - |
| NCIB 40,053 | 99,000 - |
| NCIB 40,054 | 106,000 - |
| NCIB 40,055 | 104,000 - |
| NCIB 40,056 | 101,000 - |
| NCIB 40,057 | 126,000 - |
| NCIB 40,058 | 210,000 - |
| NCIB 40,059 | 154,000 - |

These results demonstrate that the CGTases are all different.

Isoelectric Points. Isoelectric focusing employing a LKB Ampholine PAG plate pH 3.5-9.5 followed by a 0.8% Lintner starch iodine agar overlay at pH 6.0, 55°C has shown that the isoelectric points of CGTase I, II, and III are 4.55, 4.50, and 4.50, respectively.

Action Patterns. Aminex[R] HPX-42A (Bio-Rad) HPLC using refractive index for detection demonstrated that the action patterns produced from degradation of Lintner starch by each CGTase from ATCC 53,627 were identical (see Figure 4). The three CGTases are, therefore, catalytically the same. The three peaks at the right appear after hydrolysis and have been shown to be (from left to right) $\alpha$-, gamma, and $\beta$-cyclodextrin, respectively, by NMR.

Fig. 7 compares the action pattern produced by CGTase of the invention (ATCC 53,627) with a prior-art liquefying enzyme, viz. $\alpha$-amylase from Bacillus stearothermophilus.

## Conversion of Starch to Cyclodextrins.

The determination of % conversion to $\alpha$-, gamma-, or $\beta$-cyclodextrins during liquefaction with CGTase from ATCC 53,627 at a standard dose of 4.46 Phadebas U/g DS is shown below. The conditions were 35% DS corn starch at pH 4.5 with primary liquefaction at 105°C for 14 minutes and secondary liquefaction at 90°C for 4 hours. Also shown is the % conversion for CGTase (6.8 Phadebas U/g DS) with 5% DS Lintner starch - 0.1M sodium acetate (50 ppm $Ca^{2+}$) at pH 5.0 and 95°C for 50 minutes. In liquefaction equal amounts of $\alpha$- and gamma-cyclodextrins are produced while almost twice as much $\beta$-cyclodextrin is formed. In the Lintner starch reaction twice as much $\beta$-cyclodextrin is formed as gamma-cyclodextrin, however, three times as much $\alpha$-cyclodextrin is formed as gamma-cyclodextrin.

| Reaction | Cyclodextrin | | |
|---|---|---|---|
| | $\alpha$-CD | gamma-CD | $\beta$-CD |
| Lintner starch | 13.8 | 4.4 | 9.3 |
| Liquefaction | 3.8 | 3.6 | 6.4 |

## Comparison of Cyclodextrin Glycosyl Transferases.

A comparison of CGTase of the invention with published data on several known CGTases is presented in the table below. Several clear differences are evident, in particular, the temperature optimum and stability.

COMPARISON OF CYCLODEXTRIN GLYCOSYL TRANSFERASES FROM VARIOUS MICROORGANISMS

| | Invention | Bacillus stearother-mophilus | Bacillus megaterium | Bacillus circulans | Bacillus macerans | Bacillus sp. | Micrococcus sp. |
|---|---|---|---|---|---|---|---|
| | ATCC53627 | TC-60 | | ATCC9995 | IFO3490 | ATCC21783 | ATCC31606 |
| Isoelectric point | 4.50,4.55 | 4.50 | 6.07,6.80 | 5.80,6.60 | 4.60 | 5.4 | 4.20 |
| Molecular weight (SDS) | 108,000 110,000 117,000 | 68,000 | 75,000 | | 75,000 | 88,000 | 85,000 |
| Temperature optimum | 95°C (pH 5.0) | 70°C (pH 7.0) | 55°C (pH 7.0) | 55°C (pH 7.0) | 55°C (pH 6.0) | 50°C (pH 7.0) | 55-65°C (pH 7.0) |
| pH optimum | 5.0 | 6.0 | 5.2-6.2 | 7.0-9.0 | 5.2-5.7 | 4.5-5.0 & 7.5-8.5 | 5.5-6.5 & 10.0 |
| Temperature stability* (Maintains 95-100% activity) | 80°C (pH 5.0) | 50°C (pH 7.0) | 55°C (pH 7.0) | 55°C (pH 7.0) | 55°C (pH 6.0) | 60°C (pH 7.0) | 50°C (pH 7.0) |

* absence of substrate

## Starch Liquefaction Process.

Liquefaction of starch serves to partly depolymerize and solubilize starch so as to make it amenable to subsequent enzymatic saccharification, e.g. by glucoamylase. To a great extent the industry has adopted the liquefaction process of US 3,921,590. Typical conditions are heating to 105°C, e.g. by jet cooking, holding for 5 minutes at that temperature followed by a 90 minute hold at 95°C with B. licheniformis α-amylase at about pH 6. Since glucoamylase is used at a pH about 4.0-5.5, it has been desired to liquefy at a pH in this range. α-amylase from B. stearothermophilus liquefies well at pH 5.8, but both of said α-amylases are unable to liquefy below pH 5.0. The liquefaction process provided by this invention is made at about pH 4.0-5.5 (preferably 4.5-5.5), whereby subsequent saccharification can be made without intermediate pH adjustment.

CGTase of the invention does not require $Ca^{++}$ for stability, even at low pH, so addition of calcium salt is generally not needed.

Suitable liquefaction conditions are about 1-60 minutes at about 100-115°C, preferably followed by holding for about 50 minutes to 4 hours at about 80-100°C. A continuous process is preferred, and the heating is most preferably by jet-cooking. The CGTase of the invention liquefies starch well at dosage levels of 2-5 Phadebas U (see below) per gram starch DS (dry substance). The starch concentration will usually be in the range 15-40% DS (w/w% dry substance), most often 25-35% DS.

α-amylase catalyzed hydrolysis of starch results in a reduction of viscosity concomitant with an increase in reducing sugars. CGTase also degrades starch, but with essentially no generation of reducing sugars. The enzymatic reactions substantially decrease the degree of polymerization yielding a solution containing high molecular weight maltodextrins along with a substantial content of α-, β-, and gamma-cyclodextrins. Thus, the conversion of 35% DS corn starch at pH 4.5 from liquefaction at 105°C for 14 minutes and a hold period at 90°C for 4 hours into α-, β-, and gammacyclodextrin is 3.8%, 6.4%, and 3.6%, respectively.

The liquefaction process of the invention may be used for producing dextrose (glucose) in high yield from wet milled corn starch or other refined starch by liquefaction with the CGTase, followed by saccharification with glucoamylase alone or together with pullulanase.

The liquefaction process of the invention may also be used for producing ethanol from starch-containing biomass. In this case the biomass is liquefied with CGTase at a pH of 4.5-5.5, followed by saccharification with glucoamylase to form glucose and simultaneous or subsequent fermentation of the glucose to ethanol with yeast. Thereafter, the alcohol may be recovered by methods known in the art. Preferably, the whole process is carried out at pH around 5.0 without any intermediate pH adjustment, and simultaneous saccharification and fermentation is performed at about 30°C for up to 72 hours. The liquefaction can be conducted either at low DS levels (15-20%) or high DS levels (20-40%). In the high DS processes, the DS level must be reduced to about 20% prior to fermentation to obtain a maximum yeast tolerance of about 10% alcohol by volume.

The raw material for alcohol production may include refined starch such as wet milled corn starch; raw, unprocessed materials such as corn, wheat, rice, sorghum, cassawa and potato (whose starch content range from 15-80%); and other starch-containing materials such as waste and by-products from industry. In the case of refined starch, the liquefaction process preferably includes an initial treatment above 100°C followed by a hold at a lower temperature to complete liquefaction. In the case of other raw materials (with lower starch content), liquefaction is preferably carried out in the range 60-100°C.

## Production of Cyclodextrin

The present invention provides a process for producing cyclodextrins with thermostable CGTase. In the process of this invention, liquefied starch is enzymatically treated with the CGTase at a temperature exceeding about 60°C, desirably for not more than about 24 hours, and thereafter the cyclodextrins are removed from the reaction mixture.

In a preferred mode of the invention, the process involves liquefaction of a starch slurry with the CGTase enzyme at about pH 5.0 at an initial treatment temperature exceeding about 100°C followed by conversion of the liquefied starch to cyclodextrins by maintaining the liquefied starch at about 80°-90°C for up to about 24 hours in a hold step, desirably without pH adjustment, and desirably without redosing with enzyme.

In contrast to prior-art processes, the process of this invention employs temperatures sufficiently elevated to avoid serious danger of microbial contamination, which is of course advantageous. A separate (but related) advantage is that the enzymatic conversion takes place more quickly with the CGTase at the elevated conversion temperatures. Treatment time not exceeding about 24 hours in an already liquefied starch substrate is contemplated for practice of this invention. Despite the above described advantageous attributes, conversion of already liquefied starch is not a particularly preferred mode of this invention. Far more advantageous is to start the process with raw starch, e.g. a starch slurry, and use the CGTase to generate the liquefied starch therefrom.

EP 0 338 057 B1

The CGTase enzyme may be employed to liquefy starch, (i.e., generate a pourable syrup from a starch slurry) at the pH range 4.0 - 5.5 in the absence of added calcium and under standard starch liquefaction conditions (as described above). Use of the CGTase to liquefy a starch slurry and then to convert the liquefied starch into cyclodextrins constitutes a most advantageous process and constitutes the preferred practice of the invention.

Liquefaction of the starch with CGTase is accompanied by conversion of the starch into cyclodextrins. Thus, a substantial amount of cyclodextrins has been obtained and is present in the liquefied starch prior to conduct of the further enzymatic conversion of liquefied starch into cyclodextrins.

On the whole, the yield of cyclodextrins available directly from the starch liquefying process is not considered adequate. Further, enzymatic conversion of the liquefied starch with CGTase increases the yield of cyclodextrins substantially.

The elevated temperature hold treatment of the (jet) cooked starch that forms part of the standard conditions starch liquefaction process may be extended about 24 hours, desirably, however, at about 90°C for converting more of the liquefied starch into cyclodextrins, the conversion step being accompanied by modest dilution of the liquefied starch to a more optimum DS level if desired. Any pH adjustment desired for optimum enzymatic conversion into cyclodextrins may be made either on the initial starch slurry or as an incident to the dilution. However, redosing with more of the CGTase enzyme after the starch liquefaction step has not been found to be necessary.

The thermostability of the cyclodextrin glycosyl transferase of the invention which enables its use at higher enzymatic conversion temperatures than is possible for the prior art enzymes, (notably higher than for the Bacillus macerans enzyme whose limiting temperature is 50°C) allows the combined liquefaction and conversion process to be carried out without a significant intermediate cooling of the syrup. To repeat, this ability to produce cyclodextrins at elevated temperatures avoids the extended reaction times heretofore employed and in practice of this invention, reaction times of not more than about 24 hours are contemplated for the enzymatic conversion of the liquefied starch.

The starch employed in practicing the invention may be obtained from any vegetable source including, for example, waxy maize, corn, wheat, sorghum, potato, tapioca, rice, or sago. In addition to unmodified forms of the starches, modified forms derived from treatment of the starch with enzymes, acids, alkalies, etc. can also be used as substrates. The cyclodextrin production reactions can be performed on liquefied starch at DS concentrations ranging from 1% to 40% DS, but for maximum efficiency of conversion, a 20-30% DS solids solution is preferred. If desired, more concentrated starch slurries may be liquefied (standard conditions being 35% DS), then diluted to 20-30% DS dextrin solutions for conversion into cyclodextrins.

To summarize the terms of an overall process wherein the starting material is raw starch, CGTase of the invention may be employed under a wide range of conditions, including the relatively harsh standard liquefaction conditions of a 35% DS slurry, jet cooking at 105°C and 90 minute hold at 95°C over the pH 4.0-5.5 range in the absence of Ca++, followed then by a more extensive hold at about 55°C for up to about 24 hours. For maximum conversion and/or minimum CGTase usage, both hold steps (which can, of course, be a single extended hold) should be conducted within the 80°C - 90°C range, and the starch concentration be in the 20 - 30% DS range (either in the initial starch slurry or through dilution of the liquefied starch).

In further summary, the cyclodextrins can be produced from liquefied starch by incubation of the syrup with CGTase of the invention in the temperature range of 50-95°C, preferably at 80-90°C, by reacting for about 24 hours or less in the pH range 4-9 most preferably at about pH 5.0. The cyclodextrin product may be recovered from the reaction solution as heretofore. Moreover, the recovered cyclodextrins can be fractionated into $\alpha$-, $\beta$-, and gamma-cyclodextrin according to known to the art practices, e.g. through methods described by D. French et al in Journal of American Chemical Society 71:353 (1949).

The batch conversions of starch hydrolysate into cyclodextrins by the Bacillus macerans CGTase have often been performed heretofore in the presence of a suitable complexant in order to shift the equilibrium in the direction of product formation. Desirably, the cyclodextrin clathrates are insoluble and, therefore, precipitate from solution in the reaction mixture. The complexed cyclodextrin can be recovered by filtration or centrifugation, and the complexant can then be dispelled by methods known to the art. Suitable cyclodextrin complexants include cyclooctane, hexane, 1-butanol, 1-decanol, etc. A number of complexants have been identified that selectively complex with the $\alpha$- or $\beta$-form (see US Patent # 3,640,847 for example). In particular, cyclooctane is selective for $\beta$-cyclodextrin while 1-decanol is selective for $\alpha$-cyclodextrin. Practice of this invention contemplates conducting conversions using CGTase in the presence of complexants.

12

## Cyclodextrin glycosyl transferase assay

The CGTase starch-dextrinizing activity is measured by the Pharmacia Phadebas (R) Amylase Test at pH 6.0, 60°C by incubating 200 $\mu$l of the enzyme solution with 4.0 mls 0.1 M sodium acetate (100 ppm Ca$^{++}$) plus a Phadebas Tablet for 15 minutes. The reaction is then stopped with 0.5 ml 1.0 N HCl.

The assay solution is centrifuged 2 minutes in an Eppendorf centrifuge at room temperature, and the absorbance of the supernatant is read at 620 nm, an absorbance value of 1.0-3.0 should be achieved. A standard curve using Bacillus licheniformis $\alpha$-amylase as the standard was constructed where one Phadebas unit is defined as the amount of enzyme that will catalyze the hydrolysis of 1.0 $\mu$mole of glucosidic linkages of Lintner starch per minute at 60°C pH 6.0.

## Quantitation of cyclodextrin product

The yields of $\alpha$-, $\beta$-, and gamma-cyclodextrins were determined by BioRad Aminex(R) Carbohydrate HPX-42A High Performance Liquid Chromatography. Two columns (300 x 7.8 mm) were used in tandem at 85°C with glass distilled water as the eluent at a flow rate of 0.6 ml/minute. Detection was by refractive index. Standard curves were constructed with authentic samples of $\alpha$-, $\beta$-, and gamma-cyclodextrins (Sigma Chemical Company, St. Louis, Missouri).

## BRIEF DESCRIPTION OF DRAWINGS

Figure 1 is a plot of relative activity versus temperature for the CGTase;

Figure 2 is a plot of relative activity versus pH for the CGTase;

Figure 3 is a plot of the thermostability of CGTase from ATCC 53,627 relative to Bacillus stearothermophilus $\alpha$-amylase and Termamyl™;

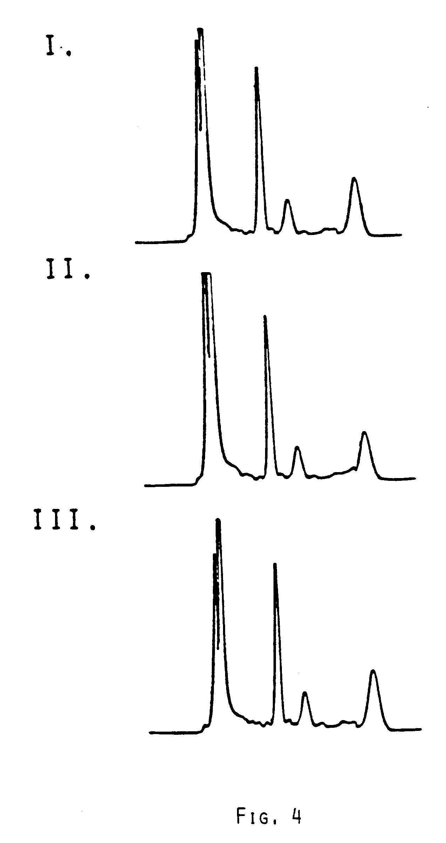

Figure 4 is the HPLC plots showing the action pattern of CGTases I, II, III;

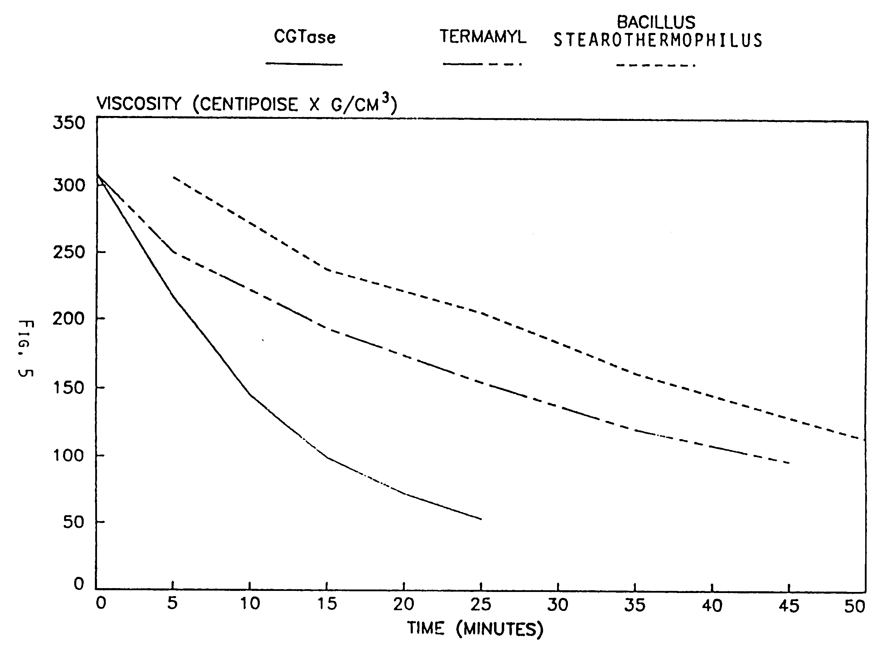

Figure 5 is a viscosity versus time plot comparing starch liquefaction with the CGTase, Termamyl™ and Bacillus stearothermophilus $\alpha$-amylase;

Figure 6 illustrates a plot of torque versus rotation speed measurement demonstrating the variations in viscosity of liquefied starch solutions obtained with varying dosage levels of CGTase;

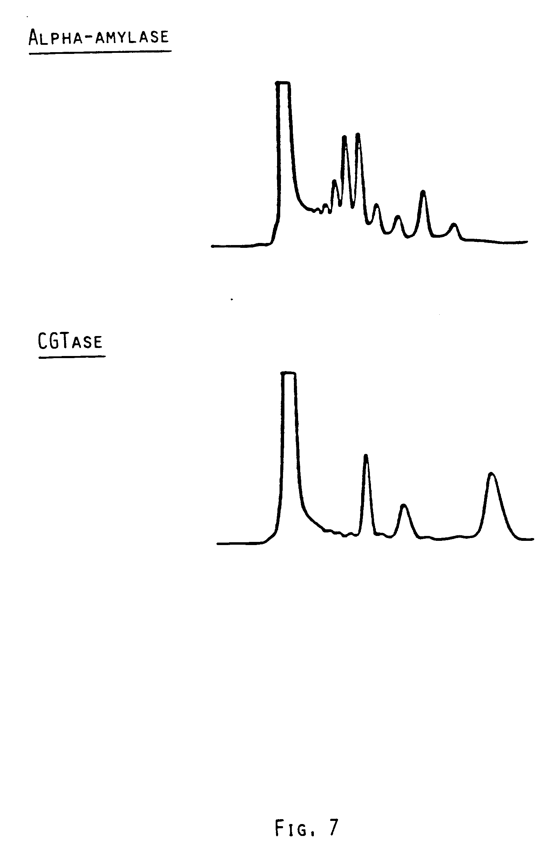

Figure 7 is the HPLC plots comparing the action patterns of the CGTase and the $\alpha$-amylase of Bacillus stearothermophilus.

## EXAMPLES

## EXAMPLE 1

### Production of CGTase by Anaerobic Cultivation

The strain ATCC 53,627 was cultured in a prereduced liquid medium under argon at an initial pH of 7 comprised of the following components in grams per litre;

Maltrin M-100, 5.0; $KH_2PO_4$, 2.0; $K_2HPO_4$, 6.0; NaCl, 1.0; $(NH_4)_2SO_4$, 2.5; $MgSO_4.7H_2O$, 0.5; $CaCl_2.2H_2O$, 0.05; yeast extract, 2.0; $Na_2S$, 0.5; cysteine-HCl, 0.5; resazurin (redox indicator), 2 ng; and trace metals 5.0 ml. The trace metals solution was comprised of the following components in grams per litre; $FeCl_3.6H_2O$, 5.40; $ZnSO_4.7H_2O$, 1.45; $MnCl_2.4H_2O$, 1.00; $CuSO_4.5H_2O$, 0.25; and $H_3BO_3$, 0.10. The trace metals solution was acidified with concentrated HCl to dissolve the salts.

The strain was incubated at 67°C without agitation for 40 hours. The maximal activity level after 40 hours was 200 Phadebas U per litre broth. The culture broth was centrifuged, then filtered, and finally concentrated to a volumetric activity of 30 - 50 Phadebas U per millilitre by use of a Millipore Minitan System.

Purification of CGTase from ATCC 53,627 was achieved by successive steps involving DEAE-Sepharose chromatography, Chromatofocusing, and acarbose-Sepharose affinity chromatography, DEAE-Sepharose chromatography was performed at pH 7.5 in 10mM Tris-HCl (2.5mM $CaCl_2$), 4°C using a linear NaCl gradient (0-200mM). Chromatofocusing (Pharmacia) was conducted at 4°C using a linear pH gradient from pH 7-5. Acarbose affinity chromatography was performed at pH 6.0, 4°C, and elution was achieved by a 0.1M sodium acetate (100 ppm Ca$^{++}$) buffer wash. Three individual CGTase components designated I, II, and III were obtained by Chromatofocusing, and purified to homogeneity by acarbose affinity chromatography. The relative amounts of the three CGTases following Chromatofocusing were CGTase I - 20%, CGTase II - 60%, and CGTase III - 20%.

**EXAMPLE 2**

Production of CGTase by Aerobic Cultivation of a Transformed Host Organism

Chromosomal DNA was isolated from cells of the strain ATCC 53,627, as follows. Cells (3.1 g wet weight) were suspended in 4.5 ml 25% sucrose - 50mM Tris pH 8.0 - 40mM EDTA. The suspension was treated with lysozyme (2 mg/ml) for 30 minutes on ice followed by 30 minutes at room temperature. Pronase (1 mg/ml) was added and the suspension was incubated at 37°C for 30 minutes. The lysate was extracted twice with 8 ml phenol for 30 minutes after adding 3 ml 10mM Tris -1mM EDTA pH 7.4 buffer. The aqueous phase was then extracted twice with 10 ml chloroform. The DNA was precipitated by adding 0.45 ml 3M sodium acetate - 1 mM EDTA pH 7.0 and 2.75 ml isopropanol and incubating on ice for 5 minutes. The DNA was pelleted by centrifugation and was washed once with 70% ethanol. The pellet was dried for 10 minutes under vacuum and then redissolved in 15 ml 10mM Tris - 1mM EDTA pH 7.4 buffer.

The DNA preparation was submitted to cesium chloride gradient centrifugation at 15°C, 192,000 g for 40 hours. The chromosomal DNA band was collected, extracted with cesium chloride saturated isopropanol three times, and dialyzed against 10mM Tris -1mM EDTA pH 7.4 buffer at 4°C. A total of 590 $\mu$g chromosomal DNA was recovered.

The DNA was partially digested with the restriction enzyme EcoRI by incubating 200 $\mu$g with 33 units of EcoRI in 150 $\mu$l of 50 mM Tris pH 8.0 - 10mM MgCl$_2$ - 100 mM NaCl for 12 minutes at 37°C. The partially digested DNA was submitted to a 10 - 40% sucrose gradient centrifugation at 135,000 g, 15°C for 16 hours. Fractions of 180 $\mu$l were collected and 5 $\mu$l aliquots were analyzed by 1% Agarose gel electrophoresis. Fractions ranging in size from 7 to 15 kb were pooled, dialyzed against 1 l 10mM Tris - 1mM EDTA pH 7.4 buffer, ethanol precipitated, and dissolved in 100 $\mu$l 10 mM Tris-1mM EDTA pH 7.4 buffer.

The vector pBR322 was digested with EcoRI by incubating 2.5 $\mu$g with 30 units of EcoRI in 125 $\mu$l of 50mM Tris pH 8.0 - 10mM MgCl$_2$ - 100mM NaCl for 2 hours at 37°C. Analysis of the DNA on an Agarose gel showed that the digestion was complete. The digested vector was extracted twice with phenol, once with ether, and ethanol precipitated.

The vector was dissolved in 100 $\mu$l of 100mM Tris pH 8.0 - 1mM MgCl$_2$ and dephosphorylated with 20 units of calf intestinal alkaline phosphatase at 37°C for 30 minutes. The dephosphorylated vector was phenol extracted twice, chloroform extracted twice, and ethanol precipitated. The dephosphorylated pBR322 was dissolved in 50 $\mu$l 10mM Tris - 1mM EDTA pH 7.4 buffer.

Ligation of the digested pBR322 and strain ATCC 53,627 DNA was accomplished by mixing 0.4 $\mu$g of the EcoRI partially digested chromosomal DNA (7-15 kb) and 0.1 $\mu$g of EcoRI digested, dephosphorylated pBR322 with 10 units T4 DNA ligase in 20 $\mu$l 50 mM Tris pH 7.4 - 10 mM MgCl$_2$ - 20mM DTT-1mM ATP containing 5 $\mu$g BSA/ml, and incubating overnight at 14°C.

Competent E. coli HB101 (ATCC 33,694) cells were prepared for transformation by the method of T. Maniatus, E.F. Fritsch, and J. Sambrook in Molecular Cloning - A Laboratory manual page 250.

One-half of the ligated DNA prepared above was transformed into competent cells of E. coli HB101 according to the method of Maniatus et al. (supra). The cells were cultured overnight at 37°C on plates containing Luria-Bertani (LB) medium and tetracycline at a concentration of 15 $\mu$g/ml. The tetracycline-resistant colonies were then replica-plated onto starch plates containing 1% amylopectin-LB medium and tetracycline (15 $\mu$g/ml), and incubated overnight at 37°C. CGTase production was determined by exposure of the starch plates after heat-treatment at 70°C for 1 hour to iodine vapor where clearing zones would be observed.

One CGTase-positive transformant designated E. coli NV601 was recovered from over 5000 colonies. The strain was both ampicillin- and tetracycline-resistant. Recovery of the recombinant plasmid by standard alkaline lysis procedures and retransformation of competent E. coli HB101 cells yielded CGTase-positive transformants.

Restriction mapping of the recombinant plasmid revealed a DNA fragment 12.8 kb in size had been inserted into the EcoRI site of pBR322. Deletion analysis with the restriction enzyme BamHI revealed that the gene encoding the CGTase was located on a 6.0 kb BamHI-BamHI fragment.

The CGTase was produced by culturing E. coli NV601 in Luria-Bertani medium containing 15 $\mu$g of tetracycline per ml medium at 37°C, 300 rpm for 24 hours. The cells were collected by centrifugation and then lysed by sonication.

Characterization of the recombinant CGTase relative to the native CGTase with respect to molecular weight (SDS-PAGE), isoelectric point, thermostability, action pattern, liquefaction activity, and cyclodextrin production indicated no difference between the enzymes. The recombinant CGTase cross-reacted with antibody raised against the native CGTase (component II).

## EXAMPLE 3

### Cyclodextrin Production

A comparison was made of the cyclodextrin yields produced by CGTase of the invention (ATCC 53,627) and the CGTase from Bacillus macerans. Since the Bacillus macerans CGTase is unable to liquefy starch under normal jet cooker conditions, a pretreated starch, i.e. Lintner starch was used. The enzymes were reacted with 15% DS Lintner starch (plus 40 ppm $Ca^{++}$) at 50°C and 90°C, pH 5.0 for 24 hours. The dosage was 4.46 Phadebas U per gram DS starch. The Bacillus macerans CGTase was also reacted at pH 7.0 as above to serve as a control.

| CGTase | pH | Temp. (°C) | Cyclodextrin Yield | | | | |
|---|---|---|---|---|---|---|---|
| | | | $\alpha$-CD % | gamma-CD % | $\beta$-CD % | Total CD, % | Total CD g/100 ml |
| Invention | 5.0 | 50 | 14.9 | 7.0 | 15.3 | 37.2 | 5.6 |
| | 5.0 | 90 | 14.9 | 6.6 | 14.6 | 36.1 | 5.4 |
| Bacillus macerans | 5.0 | 50 | 10.2 | 5.7 | 13.8 | 29.7 | 4.5 |
| | 5.0 | 90 | 0.3 | 0 | 0 | 0.3 | 0.1 |
| | 7.0 | 50 | 10.1 | 5.4 | 14.5 | 30.0 | 4.5 |
| | 7.0 | 90 | 0.5 | 0 | 0.5 | 1.0 | 0.2 |

The results demonstrate that CGTase of the invention gives superior conversion at 50°C, which is optimum for the prior-art B. macerans enzyme. The CGTase of the invention shows essentially the same high conversion at 90°C, where the prior-art enzyme is seen to be nearly inactive. The CGTase of the invention produces $\alpha$-, $\beta$- and gamma-cyclodextrin in a ratio (at 50°C) of 0.74:1.0:0.41, i.e. relatively more $\alpha$-CD than the B. macerans enzyme.

## EXAMPLE 4

### Starch Liquefaction at Various pH

35% DS corn starch with or without 40 ppm $Ca^{++}$ was liquefied at 105°C for 14 minutes followed by 4 hours at 90°C. Enzyme was dosed at 4.46 Phadebas U/g DS (60°C, pH 6.0). CGTase of the invention (ATCC 53,627) was compared with Termamyl™ (B. licheniformis $\alpha$-amylase, available from Novo Industri A/S) and B. stearothermophilus $\alpha$-amylase (available as G-Zyme™ G995 from Enzyme Bio-Systems, Ltd.).

Dextrose Equivalent (DE) was measured after liquefaction, and the starch was judged as liquefied if the starch syrup after liquefaction was pourable (indicating a substantial viscosity reduction).

EP 0 338 057 B1

| Enzyme | pH | Ca$^{++}$ | DE | Liquefied |
|---|---|---|---|---|
| CGTase | 4.5 | + | 0.51 | Yes |
| CGTase | 4.5 | - | 0.44 | Yes |
| CGTase | 5.0 | + | 0.73 | Yes |
| CGTase | 5.0 | - | 0.69 | Yes |
| CGTase | 5.5 | + | 1.10 | Yes |
| CGTase | 5.5 | - | 0.83 | Yes |
| BS Amylase | 4.5 | + | Not determinable | No |
| BS Amylase | 4.5 | - | Not determinable | No |
| BS Amylase | 5.0 | + | 4.78 | Yes* |
| BS Amylase | 5.0 | - | Not determinable | No |
| BS Amylase | 5.5 | + | 9.78 | Yes |
| BS Amylase | 5.5 | - | 5.58 | Yes |
| BS Amylase | 5.8 | + | 13.6 | Yes |
| Termamyl™ | 6.2 | + | 14.4 | Yes |

* Rated as liquefied, but very viscous.

It is seen that good liquefaction can be achieved with CGTase of the invention, even at pH as low as 4.5 without Ca$^{++}$ addition, and essentially without formation of reducing sugars.

**EXAMPLE 5**

Starch Liquefaction at Various Enzyme Dosages

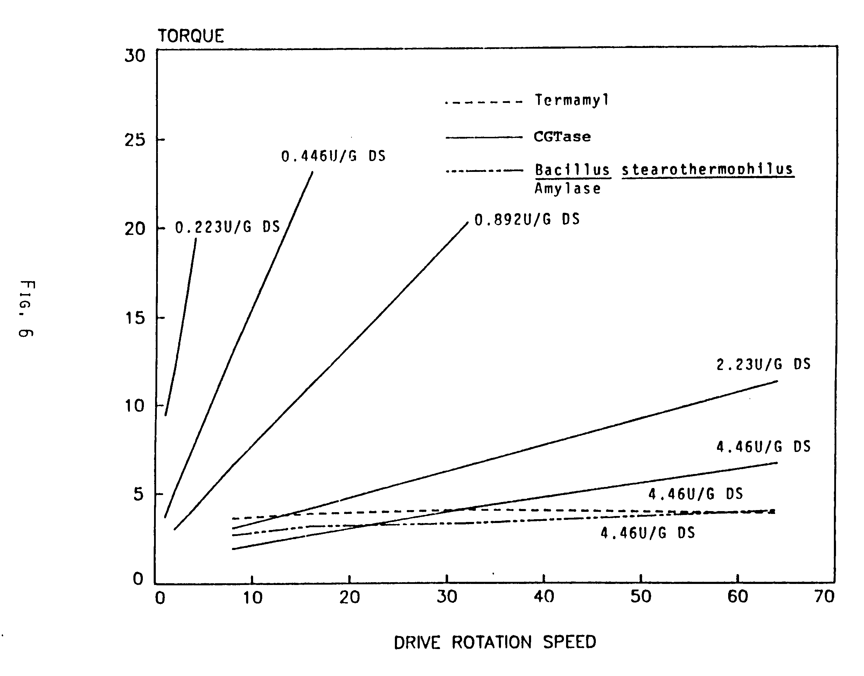

35% DS corn starch was liquefied with CGTase (ATCC 53,627) at pH 4.5 in the absence of added Ca$^{++}$. Enzyme dosages of 0.223, 0.446, 0.892, 2.23 and 4.46 Phadebas U/g DS were used. For comparison, Termamyl was used at pH 6.2 and B. stearothermophilus amylase at pH 5.8, both at 4.46 U/g DS and with 40 ppm Ca$^{++}$ present. Liquefaction conditions were 14 minutes at 100°C or 105°C (as indicated below), followed by 4 hours at 90°C. After liquefaction, viscosity was measured at 60°C with a Haake Rotovisco RV 12 viscometer with NV sensor system and M500 measuring drive unit at the respective pHs of liquefaction. Results are given below and shown in fig. 6.

| Enzyme | Dosage U/g DS | Added Ca$^{++}$ | pH | Primary Liq.temp. | Viscosity (CP) at drive rot. speed 32 |
|---|---|---|---|---|---|
| CGTase | 0.223 | - | 4.5 | 100°C | *) |
| CGTase | 0.446 | - | 4.5 | 100°C | *) |
| CGTase | 0.892 | - | 4.5 | 105°C | 208 |
| CGTase | 2.23 | - | 4.5 | 105°C | 66.9 |
| CGTase | 4.46 | - | 4.5 | 105°C | 42.4 |
| Termamyl | 4.46 | 40 ppm | 6.2 | 105°C | 41.6 |
| BS amylase | 4.46 | 40 ppm | 5.8 | 105°C | 34.1 |

*) measurement not possible at this speed

The results indicate that a dose level of 2-5 U/g DS of CGTase is suitable.

**EXAMPLE 6**

Saccharification of Liquefied Starch

The liquefied starches of example 4 were adjusted to pH 4.3 or 4.5 at 60°C, and Dextrozyme™ 150/50 was added at a dosage of 1.2 l/t DS. Dextrozyme is a mixture of glucoamylase from Aspergillus niger and pullulanase from Bacillus acidopullulyticus; it is available from Novo Industri A/S. Saccharification was performed for 48 hours at 60°C, and dextrose was determined by Bio-Rad Aminex$^R$ HPX-87C HPLC.

16

| Enzyme | Liq. pH | Sacch. pH | Ca++ | % Dextrose |
|---|---|---|---|---|
| CGTase | 4.5 | 4.5 | + | 96.0 |
| CGTase | 4.5 | 4.5 | - | 96.0 |
| CGTase | 5.0 | 4.3 | + | 95.6 |
| CGTase | 5.0 | 4.3 | - | 95.5 |
| CGTase | 5.5 | 4.3 | + | 95.7 |
| CGTase | 5.5 | 4.3 | - | 95.8 |
| BS Amylase | 4.5 | 4.3 | + | Not determinable |
| BS Amylase | 4.5 | 4.3 | - | Not determinable |
| BS Amylase | 5.0 | 4.3 | + | 96.0 |
| BS Amylase | 5.0 | 4.3 | - | Not determinable |
| BS Amylase | 5.5 | 4.3 | + | 95.8 |
| BS Amylase | 5.5 | 4.3 | - | 95.9 |
| BS Amylase | 5.8 | 4.5 | + | 96.8 |
| Termamyl™ | 6.2 | 4.5 | + | 96.4 |

The results show good saccharification of all starches liquefied according to the invention. The ability to saccharify the pH 4.5 liquefied starch demonstrates a clear process advantage of this invention over prior-art liquefactions with $\alpha$-amylase from B. lichenifornis or B. stearothermophilus since little or no pH adjustment prior to saccharification is necessary in case of starch liquefaction according to the invention.

## EXAMPLE 7

Production of Cyclodextrin at Various Starch Concentrations

The corn starch was varied from 15% to 40% DS. The slurries were first liquefied at pH 5.0 without added calcium by a 14 minute treatment at 105°C followed by a 4 hour hold at 90°C, using the CGTase (ATCC 53,627) at a dose of 4.46 Phadebas U per gram DS starch. The production of cyclodextrin was monitored after continuing the hold portion of the starch liquefaction process so as to incubate the enzyme containing dextrin solution for an additional 24 hours at pH 5.0 and 90°C. Cyclodextrin yields were determined by Bio-Rad Aminex Carbohydrate HPX-42A HPLC. Results:

| | Cyclodextrin Yield | | | | |
|---|---|---|---|---|---|
| % DS | $\alpha$-CD % | gamma-CD % | $\beta$-CD % | Total CD % | Total CD g/100 ml |
| 15 | 9.6 | 6.3 | 15.4 | 31.3 | 4.7 |
| 20 | 8.1 | 5.8 | 17.6 | 31.5 | 6.3 |
| 25 | 7.2 | 5.9 | 15.3 | 28.4 | 7.1 |
| 30 | 6.7 | 4.6 | 12.6 | 23.9 | 7.2 |
| 35 | 5.0 | 4.2 | 11.0 | 20.2 | 7.1 |
| 40 | 6.9 | 4.4 | 11.1 | 22.4 | 9.0 |

It was concluded that an initial starch concentration of about 20-30% was optimal for the production of cyclodextrins, based on Total % CD and g CD/100 ml. A concentration of 25% DS was therefore chosen for further examples. $\beta$-cyclodextrin was primarily produced at all starch concentrations. The ratio of $\beta$:$\alpha$:gamma cyclodextrins was 1.0.:0.47:0.39 at 25% DS. The highest observed yield of cyclodextrin from 25% DS starch was approximately 30%.

Redosing with the CGTase enzyme and extending the reaction time to 48 hours did not increase the yields.

## EXAMPLE 8

Production of Cyclodextrin at Various pH

The effect of pH on cyclodextrin production was determined using a 25% DS corn starch slurry. The starch slurries were liquefied at the indicated pH values, but otherwise as in Example 7, and the liquefied starches were then incubated 24 hours at 90°C at the same pH values.

The results, shown below, indicate that pH 5.0 was optimal for the combined process of starch liquefaction and cyclodextrin production.

| | Cyclodextrin Yield | | | | |
|---|---|---|---|---|---|
| pH | $\alpha$-CD % | gamma-CD % | $\beta$-CD % | Total CD % | Total CD g/100 ml |
| 4.0 | 2.5 | 1.0 | 2.0 | 5.5 | 1.4 |
| 4.5 | 5.0 | 4.3 | 8.4 | 17.7 | 4.4 |
| 5.0 | 7.4 | 5.5 | 16.7 | 29.6 | 7.4 |
| 6.0 | 8.0 | 5.3 | 14.9 | 28.2 | 7.1 |
| 7.0 | 8.4 | 5.0 | 12.9 | 26.3 | 6.6 |
| 8.0 | 8.4 | 5.0 | 13.4 | 26.8 | 6.7 |
| 9.0 | 6.3 | 4.5 | 8.5 | 19.3 | 4.8 |

## EXAMPLE 9

Production of Cyclodextrin at Various Temperatures

The effect of temperature on cyclodextrin production by CGTase of the invention (ATCC 53,627) was examined in the absence of added calcium by conduct of the incubation step for 24 hours at pH 5.0 at various temperatures, as indicated below. A 15% DS or 25% DS slurry of corn starch was first liquefied with the CGTase under the conditions of Example 7 at a dose of 4.46 Phadebas U per gram DS starch. Results:

| | 15% DS Cyclodextrin Yield | | | | |
|---|---|---|---|---|---|
| Temperature °C | $\alpha$-CD % | gamma-CD % | $\beta$-CD % | Total CD % | Total CD g/100 ml |
| 50 | 8.1 | 5.6 | 15.4 | 29.0 | 4.4 |
| 80 | 8.6 | 6.1 | 18.7 | 33.4 | 5.0 |
| 90 | 9.6 | 6.3 | 15.4 | 31.3 | 4.7 |
| 95 | 7.3 | 5.1 | 8.5 | 20.9 | 3.1 |

| | 25% DS Cyclodextrin Yield | | | | |
|---|---|---|---|---|---|
| Temperature °C | $\alpha$-CD % | gamma-CD % | $\beta$-CD % | Total CD % | Total CD g/100 ml |
| 50 | 7.1 | 4.8 | 12.1 | 24.0 | 6.0 |
| 70 | 7.2 | 5.5 | 13.7 | 26.4 | 6.6 |
| 80 | 7.2 | 5.5 | 14.6 | 27.3 | 6.8 |
| 90 | 7.2 | 5.9 | 15.3 | 28.4 | 7.1 |
| 95 | 6.3 | 5.8 | 14.6 | 26.7 | 6.7 |

The results show a conversion temperature of 80-90°C to be optimal, both at 15% DS and 25% DS (after a 24 hour incubation). Lowering the temperature to 50°C produced a lesser yield.

## EXAMPLE 10

Production of Cyclodextrin at High Temperature with and without Redosing of Enzyme.

The possibility that equilibrium had not been achieved was examined at 90°C and 95°C by redosing the reaction mixture of Example 9 involving 15% DS starch with CGTase prior to the 24 hours incubation, and allowing the reaction to continue an additional 24 hours. The results (see below) demonstrated that at 90°C equilibrium had been reached. At 95°C, redosing was necessary to achieve the same yields of cyclodextrins indicating thereby some loss of enzyme activity during prolonged incubation at 95°C.

18

| Temp. (°C) | Redose | Time (Hours) | Cyclodextrin Yield | | | | |
|---|---|---|---|---|---|---|---|
| | | | α-CD % | gamma-CD % | β-CD % | Total CD % | Total CD g/100 ml |
| 90 | - | 24 | 9.6 | 6.3 | 15.4 | 31.3 | 4.7 |
| 90 | + | 24 | 9.5 | 6.3 | 16.2 | 32.0 | 4.8 |
| 90 | - | 48 | 10.1 | 6.3 | 14.0 | 30.4 | 4.6 |
| 90 | + | 48 | 9.1 | 5.9 | 16.5 | 31.5 | 4.7 |
| 95 | - | 24 | 7.3 | 5.1 | 8.5 | 20.9 | 3.1 |
| 95 | + | 24 | 10.2 | 6.5 | 13.7 | 30.4 | 4.6 |
| 95 | - | 48 | 9.3 | 5.2 | 10.2 | 24.7 | 3.7 |
| 95 | + | 48 | 11.3 | 6.6 | 13.5 | 31.4 | 4.7 |

## EXAMPLE 11

Production of Cyclodextrin with and without Calcium Addition

The effect of calcium on cyclodextrin production was investigated. A 25% DS corn starch slurry was liquefied with CGTase of the invention (ATCC 53,627) at pH 5.0 in the presence and absence of 40 ppm calcium at a dose of 4.46 Phadebas U per gram DS starch. The liquefied starches were then incubated 24 hours at 90°C. The results (see below) indicated that presence of calcium ion had no effect on the overall yield.

| Calcium | Cyclodextrin Yield | | | | |
|---|---|---|---|---|---|
| | α-CD % | gamma-CD % | β-CD % | Total CD % | Total CD g/100 ml |
| - | 6.0 | 6.0 | 15.1 | 27.1 | 6.8 |
| + | 6.2 | 5.8 | 14.4 | 26.4 | 6.6 |

## EXAMPLE 12

Production of Cyclodextrin at Various Enzyme Dosages

The effect of varying the CGTase dose on cyclodextrin yield was determined using 25% DS corn starch. The dose was varied from 2.23 to 6.69 Phadebas U per gram DS starch. The starch was liquefied at pH 5.0 using the doses given above in the absence of added calcium with the CGTase of ATCC 53,627. The liquefied starches were then incubated 24 or 48 hours at 90°C, pH 5.0. Other conditions were as in example 5.

The results (see below) demonstrated that after 24 hours and 48 hours, the highest yields were achieved at doses of 4.46 and 3.35 Phadebas U per gram DS starch.

| Dose, Phadebas U per gram DS | Time, Hours | Cyclodextrin | | | | |
|---|---|---|---|---|---|---|
| | | α-CD % | gamma-CD % | β-CD % | Total CD % | Total CD g/100 ml |
| 2.23 | 24 | 5.8 | 5.9 | 13.0 | 24.7 | 6.2 |
| 3.35 | 24 | 6.3 | 5.8 | 14.9 | 27.0 | 6.8 |
| 4.46 | 24 | 6.0 | 6.0 | 15.1 | 27.1 | 6.8 |
| 6.69 | 24 | 5.9 | 5.2 | 10.5 | 21.6 | 5.4 |
| 2.23 | 48 | 6.7 | 6.7 | 14.8 | 28.2 | 7.1 |
| 3.35 | 48 | 6.7 | 6.5 | 15.3 | 28.5 | 7.1 |
| 4.46 | 48 | 6.7 | 6.1 | 15.8 | 28.6 | 7.1 |
| 6.69 | 48 | 6.6 | 5.6 | 13.7 | 25.9 | 6.5 |

**EXAMPLE 13**

Production of Cyclodextrin Using Complexant

The effect of cyclooctane as a $\beta$-cyclodextrin complexant in the conversion of cyclodextrins was investigated. A 25% DS corn starch slurry was liquefied at pH 5.0 in the absence of added calcium with the CGTase of ATCC 53,627 under the conditions of example 7 at a dose of 4.46 Phadebas U per gram DS starch. Then followed a conversion to cyclodextrin at 90°C for 24 hours, but cyclooctane was added at a level of 0.6 gram per gram DS starch prior to commencing the 24 hour incubation.

The conversion results (see below) demonstrated that the addition of cyclooctane increased the final cyclodextrin yield, particularly that of $\beta$-cyclodextrin.

| | Cyclodextrin Yield | | | | |
|---|---|---|---|---|---|
| | $\alpha$-CD % | gamma-CD % | $\beta$-CD % | Total CD % | Total CD g/100 ml |
| Control | 7.2 | 5.9 | 15.3 | 28.4 | 7.1 |
| With Cyclooctane: | | | | | |
| Uncomplexed | 3.7 | 2.3 | 2.6 | 8.6 | 2.2 |
| <u>Complexed</u> | 1.9 | 0.6 | 25.0 | 27.5 | 6.9 |
| Total | 5.6 | 2.9 | 27.6 | 36.1 | 9.1 |

**EXAMPLE 14**

Production of Cyclodextrin from Various Starches

A comparison of several starches was made. Slurries (25% DS) of starch from corn, potatoes, wheat, rice, and waxy maize were liquefied at pH 5.0 in the absence of added calcium with CGTase of the invention (ATCC 53,627) at a dose of 4.46 Phadebas U per gram DS starch under the conditions of Example 7. The liquefied starch solutions were then incubated 24 hours at 90°C.

The results (see below) showed that there were differences in the final cyclodextrin yield and that $\beta$-cyclodextrin was the primary product formed in all cases.

| Starch | Cyclodextrin Yield | | | | |
|---|---|---|---|---|---|
| | $\alpha$-CD % | gamma-CD % | $\beta$-CD % | Total CD % | Total CD g/100 ml |
| Corn | 7.4 | 5.4 | 15.4 | 28.2 | 7.1 |
| Potato | 9.3 | 5.6 | 14.6 | 29.5 | 7.4 |
| Wheat | 7.0 | 4.9 | 13.7 | 25.6 | 6.4 |
| Rice | 5.1 | 4.3 | 8.3 | 17.7 | 4.4 |
| Waxy Maize | 7.1 | 4.5 | 11.9 | 23.5 | 5.9 |

**EXAMPLE 15**

Ethanol Fermentation of Liquefied Starch

A 31.5% DS slurry of wet-milled corn starch was liquefied with CGTase of the invention (ATCC 53,627) at pH 5.0 without added calcium for 14 minutes at 105°C followed by 4 hours at 90°C. A control with Termamyl™ was also performed as described except at pH 6.2 in the presence of 40 ppm calcium. The enzyme dose in each case was 5.0 Phadebas units per gram DS starch.

At the end of liquefaction, the thinned starch was diluted to 22.4% DS with a yeast nutrient mix. The final concentration of the components in the nutrient mix per litre were 4.0 g yeast extract, 1.6 g ammonium phosphate, 0.4 g magnesium sulfate, 3.2 g citric acid, and 0.6 g sodium citrate. The final pH was 5.2. AMG 200 L (NOVO Laboratories, Inc., Danbury, CT) was added at a dose of 0.44% wt/wt based on the starch. Penicillin G and streptomycin sulfate were included at levels of 200 ug/ml. The fermentations were incubated at 30°C, 300 rpm for 64 hours.

The production of ethanol was indirectly measured by carbon dioxide production, i.e. weight loss as a function of time. The final ethanol yields were confirmed by Bio-Rad Aminex HPX-42A High Performance Liquid Chromatography.

After 64 hours, the yield of ethanol based on carbon dioxide production was 87.3% and 89.7% for CGTase and Termamyl liquefied starches, respectively. These yields were confirmed by Bio-Rad Aminex HPX-42A HPLC. The industrial standard yield is generally about 86 - 90%.

**EXAMPLE 16**

Production of CGTase by Anaerobic Cultivation of NCIB 40,053-40,059

Strains NCIB 40,053 through 40,053 were cultured as described in Example 1 except that cultivation temperature was 55°C.

The maximal activity level after about 40 hours of incubation in Phadebas units per litre broth was as follows: NCIB 40,053: 18, NCIB 40,054: 53, NCIB 40,055: 26, NCIB 40,056: 22, NCIB 40,057: 27, NCIB 40,058: 78, and NCIB 40,059: 10. The culture broths were centrifuged, then filtered, and finally concentrated to a volumetric activity of 30 - 50 Phadebas units per millilitre by use of a Millipore Minitan System.

**EXAMPLE 17**

Starch Liquefaction with CGTase of NCIB 40,053-40,059

The CGTase preparations prepared as in example 16 were compared as to their ability to liquefy 35% DS corn starch at pH 4.5. The starch was liquefied at 105°C for 14 minutes followed by 4 hours at 90°C at an enzyme dose of 4.46 Phadebas U/g DS (60°C, pH 6.0).

Dextrose Equivalent (DE) was measured after liquefaction, and the starch was judged as liquefied if the starch syrup after liquefaction was pourable indicating a substantial reduction in viscosity.

The results indicated that all the CGTases could achieve good liquefaction at pH 4.5 similar to the CGTase from strain ATCC 53,627. In all cases, essentially no DE was measurable indicative of CGTase activity.

Aminex[R] HPX-42A (Bio-Rad) HPLC using refractive index for detection demonstrated that the action patterns produced from liquefaction of the corn starch were typical of a CGTase where the three peaks were $\alpha$-, gamma-, and $\beta$-cyclodextrin. No major differences in the relative ratio of the cyclodextrins produced by each enzyme were observed compared to the CGTase of ATCC 53,627.

**EXAMPLE 18**

Liquefaction with Cloned CGTase

A 35% DS corn starch slurry was treated with cloned CGTase prepared as in example 2 at a dosage of 8.92 Phadebas units per g DS at pH 4.5 without added $Ca^{++}$. Jetting was done at 105°C for 5 minutes (primary liquefaction) followed by a hold at 95°C for 2 hours or 90°C for 4 hours (secondary liquefaction). During secondary liquefaction at 95°C or 90°C, a rapid reduction in viscosity was observed. At 90°C, the viscosity reduction was monitored over time using a Nametre viscometer. The results demonstrated that there was a rapid reduction in viscosity to 400 centipoise x $g/cm^3$ by 7 minutes into secondary liquefaction. The action patterns of the liquefied starches after secondary liquefaction determined by Bio-Rad Aminex[R] HPX-42A HPLC demonstrated the characteristic cyclodextrin pattern at both temperatures. DE values of < 1.0 were obtained by the neocuproine method indicating the absence of reducing end-groups consistent with the mechanism of a CGTase.

**EXAMPLE 19**

Saccharification of Starch Liquefied with Cloned CGTase

The starch solutions liquefied with CGTase at pH 4.5 in example 18 were saccharified with AMG and Dextrozyme at pH 4.5, 60°C for 48 hours at a dose of 0.18 AG per g DS. Dextrose yields were determined by Bio-Rad Aminex[(R)] HPX-87C HPLC.

| Enzyme | % Yield | | | |
|---|---|---|---|---|
| | Dextrose | DP2 | DP3 | DP4+ |
| **95°C Secondary Liquefaction** | | | | |
| Dextrozyme | 95.87 | 2.44 | 0.39 | 1.30 |
| AMG | 95.09 | 2.27 | 0.36 | 2.28 |
| **90°C Secondary Liquefaction** | | | | |
| Dextrozyme | 95.37 | 3.34 | 0.40 | 0.89 |
| AMG | 95.36 | 3.21 | 0.38 | 1.05 |

The results show a good yield of dextrose in all cases. The highest yield was achieved with secondary liquefaction at 95°C and saccharification with Dextrozyme.

**Claims**

1. A cyclodextrin glycosyl transferase, characterized in that it is native to a strain of Thermoanaerobacter or Thermoanaerobium, has a temperature optimum measured at pH 5.0 of about 95°C; a pH optimum of about 5.0; and a residual activity after 40 minutes incubation at 80°C and pH 5.0 of about 95% in the absence of starch and $Ca^{++}$.

2. A cyclodextrin glycosyl transferase according to Claim 1 native to strain ATCC 53,627 or one of the strains NCIB 40,053 through NCIB 40,059.

3. A method for producing a cyclodextrin glycosyl transferase (CGTase), which has a temperature optimum measured at pH 5.0 of about 95°C; a pH optimum of about 5.0; and a residual activity after 40 minutes incubation at 80°C and pH 5.0 of about 95% in the absence of starch and $Ca^{++}$, comprising cultivation of a CGTase producing strain of Thermoanaerobacter or Thermoanaerobium under anaerobic conditions, or cultivation of a transformed host organism containing the appropriate genetic information therefrom under aerobic conditions, in a suitable nutrient containing medium and thereafter recovering CGTase from the fermentation medium.

4. A method according to Claim 3, wherein the strain is ATCC 53,627, one of the strains NCIB 40,053 through NCIB 40,059 or a mutant or variant thereof capable of producing a cyclodextrin glycosyl transferase with the characteristics indicated in Claim 1.

5. A method according to claim 3, wherein the host organism is a strain of Escherichia, Streptomyces, Bacillus or Aspergillus, preferably a strain of E.coli, B. subtilis, B. licheniformis or A. oryzae.

6. A biologically pure culture of a strain of Thermoanaerobacter or Thermoanaerobium, characterized by the ability to produce cyclodextrin glycosyl transferase, which has a temperature optimum measured at pH 5.0 of about 95°C; a pH optimum of about 5.0; and a residual activity after 40 minutes incubation at 80°C and pH 5.0 of about 95% in the absence of starch and $Ca^{++}$, and by being non-motile.

7. A culture according to Claim 6 of the strain ATCC 53,627, one of the strains NCIB 40,053 through NCIB 40,059 or a mutant or variant thereof capable of producing a cyclodextrin glycosyl transferase with the characteristics indicated in Claim 1.

8. A starch liquefaction process which comprises subjecting an aqueous starch slurry to enzymatic liquefaction in the presence of the CGTase of Claim 1 or 2 at a pH in the range of about 4.0 to 5.5 preferably at a temperature exceeding about 100°C.

9. A starch liquefaction process according to Claim 8 essentially without addition of a calcium salt to the starch slurry.

10. A process according to Claim 8 or 9, whereby the liquefied starch is thereafter subjected to enzymatic saccharification in the presence of glucoamylase, substantially without an intermediate pH adjustment.

11. A process according to Claim 10 further comprising ethanol fermentation with yeast simultaneously with or subsequent to said saccharification.

12. A process for producing cyclodextrin which comprises enzymatically treating a liquefied starch solution with the cyclodextrin glycosyl transferase of Claim 1, at a temperature of above 60°C and thereafter recovering a cyclodextrin product from the reaction mixture.

13. A process according to Claim 12, wherein said enzyme treatment is carried out for less than about 24 hours.

14. A process according to Claim 12 or 13, wherein said liquefied starch solution is generated by enzymatically liquefying a starch slurry with said CGTase.

15. A process according to Claim 14, wherein said enzyme liquefaction is done at a pH in the range of about 4.0 - 5.5, and said subsequent enzyme treatment of the starch hydrolyzate is done substantially without intermediate pH adjustment.

16. A process according to Claim 14 or 15, wherein said enzyme treatment of the starch hydrolyzate is done without redosing the liquefied starch with CGTase.

17. A process for producing cyclodextrin which comprises enzymatically treating an aqueous starch slurry with the cyclodextrin glycosyl transferase of Claim 1 at a temperature of above about 100°C and at a pH in the range of 4.0 - 5.5, preferably essentially without addition of a calcium salt, thereafter holding the resulting syrup at a temperature in the range of 80° - 90°C for not more than about 28 hours, the syrup being in the range of 20 - 30 DS during at least part of said hold period, and then recovering a cyclodextrin product from the reaction mixture.

**Patentansprüche**

1. Cyclodextrin-Glycosyl-Transferase, dadurch gekennzeichnet, daß sie für einen Stamm von Thermoanaerobacter oder Thermanaerobium nativ ist, ein Temperatur-Optimum, gemessen bei pH 5,0, von etwa 95°C; ein pH-Optimum von etwa 5,0; und eine Restaktivität nach 40 Minuten Inkubation bei 80°C und pH 5,0 von etwa 95 % in der Abwesenheit von Stärke und Ca$^{++}$ besitzt.

2. Cyclodextrin-Glycosyl-Transferase nach Anspruch 1, die für Stamm ATCC 53,627 oder einen der Stämme NCIB 40,053 bis NCIB 40,059 nativ ist.

3. Verfahren zur Herstellung einer Cyclodextrin-Glycosyl-Transferase (CGTase), die ein Temperatur-Optimum, gemessen bei 5,0, von etwa 95°C besitzt; ein pH-Optimum von etwa 5,0; und eine Restaktivität nach 40 Minuten Inkubation bei 80°C und pH 5,0 von etwa 95 % in der Abwesenheit von Stärke und Ca$^{++}$ besitzt, wobei das Verfahren Kultivierung eines CGTase produzierenden Stammes von Thermoanaerobacter oder Thermoanaerobium unter anaeroben Bedingungen oder Kultivierung eines transformierten Wirtsorganismus, der die passende genetische Information daraus enthält, unter aeroben Bedingungen in einem geeigneten nährstoffhaltigen Medium und danach Gewinnung von CGTase

EP 0 338 057 B1

aus dem Fermentationsmedium umfaßt.

4. Verfahren nach Anspruch 3, wobei der Stamm ATCC 53,627, einer der Stämme NCIB 40,053 bis NCIB 40,059 oder eine Mutante oder Variante derselben ist, der (die) eine Cyclodextrin-Glycosyl-Transferase mit den in Anspruch 1 angegebenen Merkmalen produzieren kann.

5. Verfahren nach Anspruch 3, wobei der Wirtsorganismus ein Stamm von Escherichia, Streptomyces, Bacillus oder Aspergillus ist, vorzugsweise ein Stamm von E.coli, B. subtilis, B. licheniformis oder A. oryzae.

6. Biologisch reine Kultur eines Stammes von Thermoanaerobacter oder Thermoanaerobium, gekennzeichnet durch die Fähigkeit, Cyclodextrin-Glycosyl-Transferase zu produzieren, die ein Temperatur-Optimum, gemessen bei pH 5,0 von etwa 95 °C; ein pH-Optimum von etwa 5,0; und eine Restaktivität nach 40 Minuten Inkubation bei 80 °C und pH 5,0 von etwa 95 % in der Abwesenheit von Stärke und Ca$^{++}$ besitzt, und dadurch, daß sie nicht freibeweglich ist.

7. Kultur nach Anspruch 6 des Stammes ATCC 53,627, eines der Stämme NCIB 40,053 bis NCIB 40,059 oder einer Mutante oder Variante derselben, der (die) eine Cyclodextrin-Glycosyl-Transferase mit den in Anspruch 1 angegebenen Merkmalen produzieren kann.

8. Stärkeverflüssigungsverfahren, welches umfaßt, daß eine wäßrige Stärkeaufschlämmung enzymatischer Verflüssigung in der Gegenwart der CGTase von Anspruch 1 oder 2 bei einem pH im Bereich von etwa 4,0 bis 5,5 vorzugsweise bei einer Temperatur, die etwa 100 °C übersteigt, unterzogen wird.

9. Stärkeverflüssigungsverfahren nach Anspruch 8, im wesentlichen ohne Zugabe eines Calciumsalzes zur Stärkeaufschlämmung.

10. Verfahren nach Anspruch 8 oder 9, wobei die verflüssigte Stärke anschließend enzymatischer Verzukkerung in der Gegenwart von Glucoamylase unterzogen wird, im wesentlichen ohne eine pH-Zwischeneinstellung.

11. Verfahren nach Anspruch 10, das außerdem Ethanol-Fermentation mit Hefe gleichzeitig mit oder anschließend an besagte Verzuckerung umfaßt.

12. Verfahren zur Herstellung von Cyclodextrin, das enzymatische Behandlung einer verflüssigten Stärkelösung mit der Cyclodextrin-Glycosyl-Transferase von Anspruch 1 bei einer Temperatur von über 60 °C und danach Gewinnung eines Cyclodextrin-Produktes aus der Reaktionsmischung umfaßt.

13. Verfahren nach Anspruch 12, wobei besagte Enzymbehandlung für weniger als etwa 24 Stunden durchgeführt wird.

14. Verfahren nach Anspruch 12 oder 13, wobei besagte verflüssigte Stärkelösung durch enzymatisches Verflüssigen einer Stärkeaufschlämmung mit besagter CGTase erzeugt wird.

15. Verfahren nach Anspruch 14, wobei besagte Enzymverflüssigung bei einem pH im Bereich von etwa 4,0 - 5,5 durchgeführt wird und besagte anschließende Enzymbehandlung des Stärkehydrolysats im wesentlichen ohne pH-Zwischeneinstellung durchgeführt wird.

16. Verfahren nach Anspruch 14 oder 15, wobei besagte Enzymbehandlung des Stärkehydrolysats ohne erneute Dosierung der verflüssigten Stärke mit CGTase durchgeführt wird.

17. Verfahren zur Herstellung von Cyclodextrin, welches umfaßt, daß eine wäßrige Stärkeaufschlämmung mit der Cyclodextrin-Glycosyl-Transferase von Anspruch 1 bei einer Temperatur von über etwa 100 °C und einem pH im Bereich von 4,0 - 5,5, vorzugsweise im wesentlichen ohne Zugabe eines Calciumsalzes, enzymatisch behandelt wird, der resultierende Sirup danach bei einer Temperatur im Bereich von 80° - 90 °C für nicht mehr als etwa 28 Stunden gehalten wird, wobei der Sirup während wenigstens eines Teils besagter Halteperiode im Bereich 20 - 30 TS liegt, und dann ein Cyclodextrin-Produkt aus der Reaktionsmischung gewonnen wird.

24

**Revendications**

1. Cyclodextrine glycosyltransférase, caractérisée en ce qu'elle est issue d'une souche de Thermoanaerobacter ou Thermoanaerobium, a un optimum de température d'environ 95°C mesuré à un pH de 5,0; un optimum de pH d'environ 5,0; et une activité résiduelle d'environ 95 % au bout de 40 minutes d'incubation à 80°C et à un pH de 5,0 en l'absence d'amidon et de Ca⁺⁺.

2. Cyclodextrine glycosyltransférase selon la revendication 1, issue de la souche ATCC 53 627 ou de l'une des souches NCIB 40 053 à NCIB 40 059.

3. Méthode de production d'une cyclodextrine glycosyltransférase (CGTase), qui a un optimum de température d'environ 95°C mesuré à un pH de 5,0; un optimum de pH d'environ 5,0; et une activité résiduelle d'environ 95 % au bout de 40 minutes d'incubation à 80°C et à un pH de 5,0 en l'absence d'amidon et de Ca⁺⁺, cette méthode comprenant la culture dans des conditions anaérobies d'une souche de Thermoanaerobacter ou Thermoanaerobium productrice de CGTase, ou la culture dans des conditions aérobies d'un organisme hôte transformé contenant l'information génétique appropriée, dans un milieu nutritif convenable, puis la récupération de la CGTase à partir du milieu de fermentation.

4. Méthode selon la revendication 3, dans laquelle la souche est la souche ATCC 53 627, l'une des souches NCIB 40 053 à NCIB 40 059, ou un de leurs mutants ou une de leurs variantes capables de produire une cyclodextrine glycosyltransférase ayant les caractéristiques indiquées dans la revendication 1.

5. Méthode selon la revendication 3, dans laquelle l'organisme hôte est une souche d'Escherischia, de Streptomyces, de Bacillus ou d'Aspergillus, de préférence une souche d'E. coli, de B. subtilis, de B. licheniformis ou d'A. orizae.

6. Culture biologiquement pure d'une souche de Thermoanaerobacter ou Thermoanaerobium, caractérisée par sa capacité à produire une cyclodextrine glycosyltransférase qui a un optimum de température d'environ 95°C mesuré à un pH de 5,0; un optimum de pH d'environ 5,0; et une activité résiduelle d'environ 95 % au bout de 40 minutes d'incubation à 80°C et à un pH de 5,0 en l'absence d'amidon et de Ca⁺⁺, et par son absence de mobilité.

7. Culture selon la revendication 6 de la souche ATCC 53 627, de l'une des souches NCIB 40 053 à NCIB 40 059, ou d'un de leurs mutants ou d'une de leurs variantes capables de produire une cyclodextrine glycosyltransférase ayant les caractéristiques indiquées dans la revendication 1.

8. Procédé de liquéfaction d'amidon dans lequel on soumet une suspension d'amidon aqueuse à une liquéfaction enzymatique en présence de la CGTase de la revendication 1 ou 2 à un pH compris entre environ 4,0 et 5,5, de préférence à une température supérieure à environ 100°C.

9. Procédé de liquéfaction d'amidon selon la revendication 8, effectué essentiellement sans addition de sel de calcium à la pâte d'amidon.

10. Procédé selon la revendication 8 ou 9, dans lequel l'amidon liquéfié est ensuite soumis à une saccharification enzymatique en présence de glùcoamylase, essentiellement sans ajustement du pH intermédiaire.

11. Procédé selon la revendication 10, comprenant en outre une fermentation éthanolique avec une levure, simultanée ou postérieure à ladite saccharification.

12. Procédé de production de cyclodextrine, qui comprend le traitement enzymatique d'une solution d'amidon liquéfié avec la cyclodextrine glycosyltransférase de la revendication 1, à une température supérieure à 60°C, puis la récupération de la cyclodextrine produite à partir du mélange de réaction.

13. Procédé selon la revendication 12, dans lequel ledit traitement enzymatique est effectué pendant moins de 24 heures environ.

**14.** Procédé selon la revendication 12 ou 13, dans lequel ladite solution d'amidon liquéfié est produite par liquéfaction enzymatique d'une suspension d'amidon avec ladite CGTase.

**15.** Procédé selon la revendication 14, dans lequel ladite liquéfaction enzymatique est effectuée à un pH compris entre environ 4,0 et 5,5, et ledit traitement enzymatique ultérieur de l'hydrolysat d'amidon est effectué essentiellement sans ajustement du pH intermédiaire.

**16.** Procédé selon la revendication 14 ou 15, dans lequel ledit traitement enzymatique de l'hydrolysat d'amidon est effectué sans nouvelle addition de CGTase à l'amidon liquéfié.

**17.** Procédé de production de cyclodextrine qui comprend le traitement enzymatique d'une suspension d'amidon aqueuse avec la cyclodextrine glycosyltransférase de la revendication 1 à une température supérieure à environ 100°C et à un pH compris entre 4,0 et 5,5, de préférence essentiellement sans addition de sel de calcium, suivi du maintien du sirop obtenu à une température comprise entre 80 et 90°C pendant au plus 28 heures, le sirop ayant une concentration comprise entre 20 et 30 % de MS (matière sèche) pendant au moins une partie de ladite période de maintien, puis la récupération de la cyclodextrine produite à partir du mélange réactionnel.

% RELATIVE ACTIVITY

FIG. 1

TEMPERATURE °C

EP 0 338 057 B1

27

% ACTIVITY

Fig. 2

pH

EP 0 338 057 B1

FIG. 3

% Residual Activity

Temperature (°C)

CGTase    Bacillus stearothermophilus    Termamyl

EP 0 338 057 B1

29

I.

II.

III.

FIG. 4

CGTase — TERMAMYL — BACILLUS STEAROTHERMOPHILUS

VISCOSITY (CENTIPOISE X G/CM$^3$)

FIG. 5

TIME (MINUTES)

EP 0 338 057 B1

31

Fig. 6

EP 0 338 057 B1

32

TORQUE

0.446U/G DS

0.223U/G DS

0.892U/G DS

Termamyl

CGTase

Bacillus stearothermophilus
Amylase

2.23U/G DS

4.46U/G DS

4.46U/G DS

4.46U/G DS

DRIVE ROTATION SPEED

ALPHA-AMYLASE

CGTASE

FIG. 7